# EUROPEAN PATENT APPLICATION

(11) **EP 3 836 009 A1**
(43) Date of publication of application: **16.06.2021**
(21) Application number: 20166098.2
(22) Date of filing: 26.03.2020
(51) Int. Cl.: G06K 9/00, G06K 9/52

(54) **METHOD FOR ANALYZING AND EVALUATING FACIAL MUSCLE STATUS**

(30) Priority: 09.12.2019 TW 108144947
(71) Applicant: CAL-COMP BIG DATA, INC, Shenkeng, New Taipei City 222 (TW)
(72) Inventor: WANG, Hong-Chun, New Taipei City 222 (TW)
(74) Representative: 2K Patentanwälte Blasberg Kewitz & Reichel

(57) **Abstract**

A method for analyzing and evaluating facial muscle status includes following steps: capturing user's (2) face image through an image capturing unit (12) of a face image analyzing apparatus (1) after it is activated; analyzing the face image through an analyzing algorithm (151) for obtaining multiple ideal muscle identifying points (41-44) corresponding to five sense features of a face in the face image; identifying the face image through a fuzzy comparison algorithm (152) and a training model (153) for obtaining multiple actual muscle identifying points (21-24) corresponding to actual muscle status of the face in the face image; evaluating each of the actual muscle identifying points (21-24) and generating evaluated results based on the multiple ideal muscle identifying points (41-44) in company with a pre-stored evaluation rule; and, displaying the multiple ideal muscle identifying points (41-44), the multiple actual muscle identifying points (21-24) and the evaluated results on a display (11) of the face image analyzing apparatus.

## Description

### BACKGROUND OF THE INVENTION

### 1. Technical Field

The invention relates to a method for analyzing and evaluating a face, and specifically to a method for analyzing and evaluating the facial muscle status of the face.

### 2.Description of Related Art

Human's muscle (especially facial muscle) will slowly slacken and droop while people age, and parts of the users choose to use care products to maintain their muscle and skin, such as use cosmetics to cover the slackened muscle, or go to work out for slowing down the speed of muscle slackening.

General speaking, users will be sitting in front of the mirror for using the care products and/or the cosmetics, or using the care products and/or the cosmetics through the assistance of smart phones, laptops or special makeup assisting devices in order to improve the speed and quality of using the same.

However, such devices can only assist the users in using the care products/cosmetics, but it is incapable of actively analyzing user's muscle status. Therefore, users cannot be aware of whether the care products/cosmetics they have been using are effective after using it for a period of time. Besides, even if the users constantly do the exercise or go to the aesthetic medicine clinic for miro plastic surgery, users can only use bare eyes to determine their skin condition by their own, but still they cannot ensure whether the execise or miro plastic surgery they did are really helping their skin.

According to the above problem, a novel analyzing and evaluating method should be provied in this field for effectively analyzing and evaluating user's current muscle status, so the user can easily realize whether the currently applied maintenance manners are effective.

### SUMMARY OF THE INVENTION

The invention is directed to a method for analyzing and evaluating facial muscle status of a face, which can obtain multiple ideal muscle identifying points as well as multiple actual muscle identifying points from user's face after analyzing the face, so as to evaluate user's current facial muscle status.

In one of the exemplary embodiments, the method of the present invention is applied to a face image analyzing apparatus and includes following steps: capturing user's face image through an image capturing unit of the face image analyzing apparatus; analyzing the face image through an analyzing algorithm for obtaining multiple ideal muscle identifying points corresponding to the frame of user's face in the face image; identifying the face image through a fuzzy comparison algorithm and a training model for obtaining multiple actual muscle identifying points corresponding to actual muscle status of user's face; evaluating each of the actual muscle identifying points based on the multiple ideal muscle identifying points in company with a pre-stored evaluation rule; and, displaying the multiple ideal muscle identifying points, the multiple actual muscle identifying points and evaluated results on a display of the face image analyzing apparatus.

In comparison with related art, the disclosures of the present invention can analyze and evaluate user's face through comparing multiple ideal muscle identifying points with multiple actual muscle identifying points upon user's face, so as to assist the user to be aware of his/her current facial muscle status in order to ensure whether the currently applied maintenance manners are effective or not.

### BRIEF DESCRIPTION OF THE DRAWINGS

FIG. 1 is a schematic diagram of a face image analyzing apparatus according to a first embodiment of the present invention.
FIG. 2 is a block diagram of the face image analyzing apparatus according to a first embodiment of the present invention.
FIG. 3 is a schematic diagram showing multiple actual muscle identifying points according to a first embodiment of the present invention.
FIG. 4 is an evaluating flowchart according to a first embodiment of the present invention.
FIG. 5 is a schematic diagram showing the comparison of multiple actual muscle identifying points and multiple ideal muscle identifying points according to a first embodiment of the present invention.
FIG. 6 is a flowchart for analyzing multiple ideal muscle identifying points according to a first embodiment of the present invention.
FIG. 7 is a schematic diagram showing multiple ideal muscle identifying points according to a first embodiment of the present invention.
FIG. 8 is a schematic diagram showing an evaluation rule according to a first embodiment of the present invention.
FIG. 9 is a schematic diagram showing an evaluation rule according to a second embodiment of the present invention.
FIG. 10 is a schematic diagram showing an evaluated result according to a first embodiment of the present invention.

### DETAILED DESCRIPTION OF THE INVENTION

In cooperation with the attached drawings, the technical contents and detailed description of the present invention are described thereinafter according to multiple embodiments, being not used to limit its executing scope. Any equivalent variation and modification made according to appended claims is all covered by the claims claimed by the present invention.

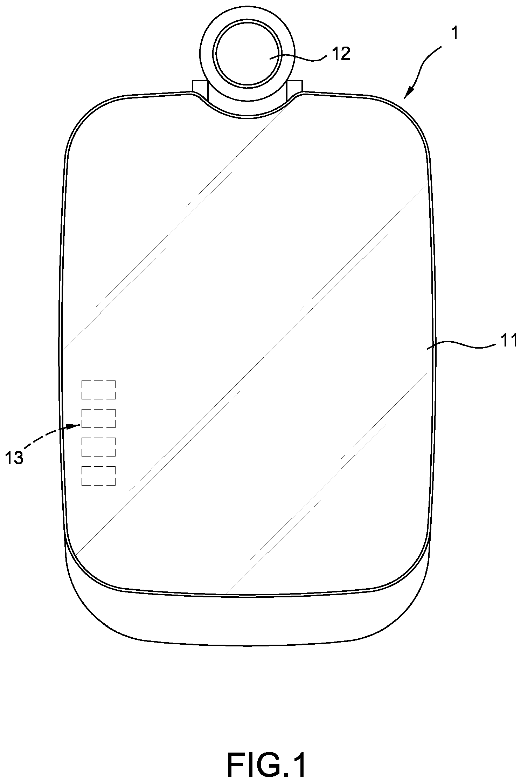

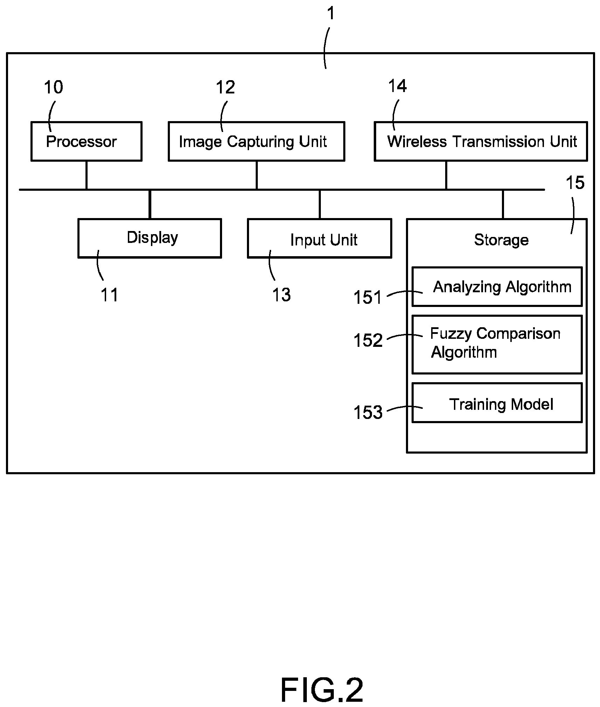

FIG. 1 is a schematic diagram of a face image analyzing apparatus according to a first embodiment of the present invention. FIG. 2 is a block diagram of the face image analyzing apparatus according to a first embodiment of the present invention.

The present invention discloses a method for analyzing and evaluating facial muscle status (referred to as the evaluating method hereinafter), and the evaluating method is mainly applied to a face image analyzing apparatus 1 (referred to as the analyzing apparatus 1 hereinafter) as shown in FIG. 1 and FIG. 2. In particular, one of the main technical feature of the present invention utilizes such analyzing apparatus 1 to obtain one or more features related to user's facial muscle and analyze the same, so as to evaluate user's current facial muscle status based on these features. As a result, user may easily understand his/her current facial muscle status, and he or she can estimate whether the currently applied maintenance manners, such as care products, cosmetics, exercises, miro plastic surgery, are effective or not.

The analyzing apparatus 1 shown in FIG. 1 and FIG. 2 is used to assist the user to make up, and the analyzing apparatus 1 can analyze and evaluate user's facial status before and after making up. It is worth saying that if same or similar hardware components of the analyzing apparatus 1 are existed in other electronic devices such as smart phones, laptops, etc. and corresponding applications are installed in such electronic devices, the evaluating method of the present invention can be accomplished the same on such electronic devices, and it is unnecessarily limited and restricted only on the analyzing apparatus 1 shown in FIG. 1 and FIG. 2 being discussed in the following.

As shown in FIG. 1 and FIG. 2, the analyzing apparatus 1 mainly includes a processor 10, a display 11, an image capturing unit 12, an input unit 13, a wireless transmission unit 14, and a storage 15. The processor 10 is electrically connected with the display 11, the image capturing unit 12, the input unit 13, the wireless transmission unit 14, and the storage 15 through serial buses for integrating and controlling these components.

The analyzing apparatus 1 can capture a photo of the user (especially a photo includes the user's face) through the image capturing unit 12, and retrieve a face image of the user from the photo and displays the face image through the display 11. Also, the analyzing apparatus 1 may display guidance information (for example, directly labelling each makeup area on the face image to indicate the user which cosmetic to use, or utilizing words or pictures to provide makeup steps or suggestions to the user) on the display 11. Therefore, the user can easily perform his/her makeup or maintenance according to the assistance from the analyzing apparatus 1.

The input unit 13 is arranged at one side of the analyzing apparatus 1, and can be a physical style unit or a touch style unit. By using the input unit 13, the user is allowed to interact with the analyzing apparatus 1, so as to operate the analyzing apparatus 1 and instruct the same. For example, the user can select different makeup areas (such as blush areas, foundation areas, etc.) on the face image to be labeled by the analyzing apparatus 1, or switch the makeup steps/makeup suggestions provided by the analyzing apparatus 1 (such as page-up, page-down, etc.).

In one embodiment, the display 11 is a touch screen which can be operated directly by the user. In this embodiment, the input unit 13 and the display 11 are integrated into one component and not existed individually.

The wireless transmission unit 14 is utilized to connect to the Internet, so the analyzing apparatus 1 can connect to a remote electronic device or server through the Internet. In the present invention, the analyzing apparatus 1 utilizes one or more algorithms to analyze and evaluate the user's facial muscle status of the face in the face image, a d those algorithms and corresponding database can be alternatively stored in the analyzing apparatus 1 or the remote electronic device/server, not limited thereto. Besides, the user can operate a user terminal (not shown) to connect to the analyzing apparatus 1 through network, so as to perform firmware maintenance and firmware updating to the analyzing apparatus 1 from a remote place.

In one embodiment, the analyzing apparatus 1 captures user's face image in real-time through the image capturing unit 12, and analyzes the face image for evaluating user's current facial muscle status. In other embodiment, the analyzing apparatus 1 downloads user's face image pre-shot before from the remote electronic device or server through the Internet, and analyzes facial features in the face image for evaluating user's facial muscle status back at the time the user took this face image.

The storage 15 stores algorithms and database(s) utilized by the analyzing apparatus 1 in performing the evaluating method of the present invention. In particular, the storage 15 stores at least an analyzing algorithm 151, a fuzzy comparison algorithm 152, and a training model 153, but not limited thereto. As discussed above, the analyzing algorithm 151, the fuzzy comparison algorithm 152, and the training model 153 can also be stored in the remote electronic device or remote server, and the analyzing apparatus 1 can access the remote electronic device or the remote server through the Internet for executing the analyzing algorithm 151, the fuzzy comparison algorithm 152, and the training model 153 remotely existed in order to implement the evaluating method of the present invention.

In other embodiment, the analyzing algorithm 151 and the fuzzy comparison algorithm 152 can be embedded in the processor 10 as a part of firmware of the processor 10, but not limited thereto.

In the present invention, a manufacturer of the analyzing apparatus 1 can pre-import a bunch of unspecified face images into a database, and manually labels multiple facial muscle identifying points defined by professors (such as doctors, cosmetologist, etc.) respectively on each of the face images, so as to train and create the aforementioned training model 153. In one embodiment, the multiple facial muscle identifying points manually labeled on each of the face images are respectively corresponding to the positions of risorius muscle and masticatory muscle of a face covered in the face image.

One of the main technical features of the present invention is that, when obtaining a new face image, the analyzing apparatus 1 first performs an image analysis process to the face image through an algorithm for calculating the positions that multiple facial muscle identifying points supposed to be (i.e., the ideal positions) on a face in the face image according to the positions of five sense features of the face. Also, the analyzing apparatus 1 imports the same face image into the training model 153 for identifying the positions that multiple facial muscle identifying points actually exist (i.e., the actual positions) on the face in the face image. Therefore, the analyzing apparatus 1 is able to use the difference between the ideal positions and the actual positions of the multiple facial muscle identifying points to evaluate the muscle status of the face covered in the face image.

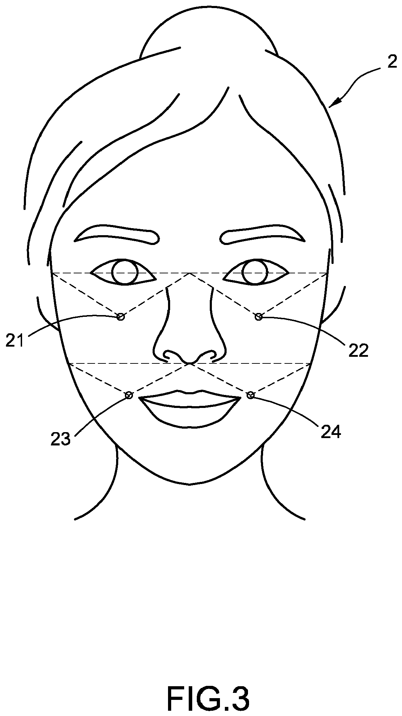

Refers to FIG. 3, which is a schematic diagram showing multiple actual muscle identifying points according to a first embodiment of the present invention. As shown in FIG. 3, after obtaining a photo of a user 2 and retrieving a facial image from the photo, the analyzing apparatus 1 identifies the facial image by way of the pre-trained training model 153, so as to find the actual positions of multiple muscle identifying points on a face covered by the facial image. The multiple muscle identifying points located at those actual positions are referred to as multiple actual muscle identifying points in the following description.

In the embodiment of FIG. 3, the multiple actual muscle identifying points include a first actual muscle identifying point 21 corresponding to an actual position of right side risorius muscle of the face in the face image, a second actual muscle identifying point 22 corresponding to an actual position of left side risorius muscle of the face in the face image, a third actual muscle identifying point 23 corresponding to an actual position of right side masticatory muscle of the face in the face image, and a fourth actual muscle identifying point 24 corresponding to an actual position of left side masticatory muscle of the face in the face image.

Within a certain range, the muscle status of the user 2 is determined better (for example, younger or tighter) if the positions of the first actual muscle identifying point 21 and the second actual muscle identifying point 22 are close to the inner side of the face (e.g., close to the nose) as well as the upper side of the face (e.g., close to the eyes). In one embodiment, the analyzing apparatus 1 can simulate a first virtual triangle constituted by the first actual muscle identifying point 21, a position of nasion, and a position of right temple as well as a second virtual triangle constituted by the second actual muscle identifying point 22, the position of nasion, and a position of right temple. According to the aforementioned disclosure, within a certain range, the muscle status of the user 2 is determined better once the square measures of the first and second virtual triangles are smaller, in other words, the square measures of the first and second virtual triangles are inversely proportional to the muscle status of the user 2.

Similarly, within a certain range, the muscle status of the user 2 is determined better if the positions of the third actual muscle identifying point 23 and the fourth actual muscle identifying point 24 are close to the inner side of the face (e.g., close to the mouse) as well as the upper side of the face (e.g., close to the nose). In one embodiment, the analyzing apparatus 1 can simulate a third virtual triangle constituted by the third actual muscle identifying point 23, a position of nose tip, and a position of right cheek as well as a fourth virtual triangle constituted by the fourth actual muscle identifying point 24, the position of nose tip, and a position of left cheek. Accordingly, within a certain range, the muscle status of the user 2 is determined better once the square measures of the third and fourth virtual triangles are smaller, in other words, the square measures of the third and fourth virtual triangles are also inversely proportional to the muscle status of the user 2.

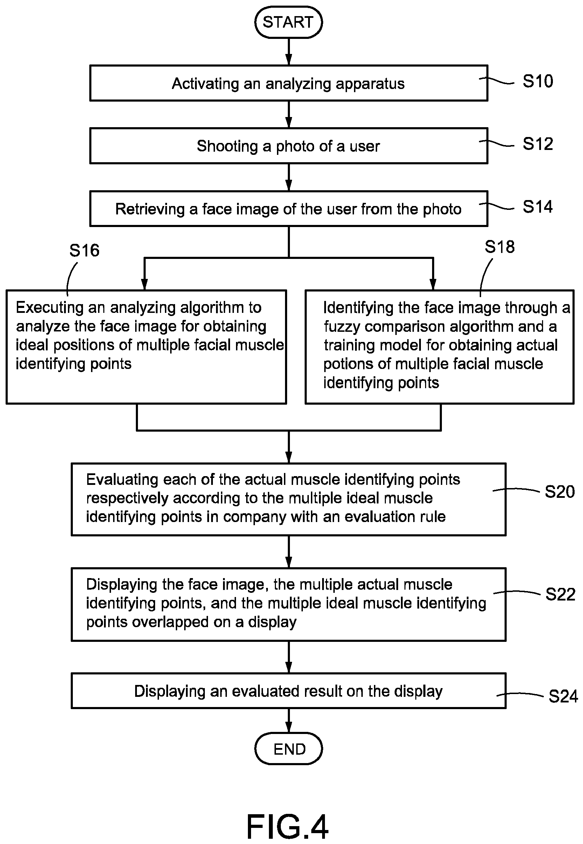

FIG. 4 is an evaluating flowchart according to a first embodiment of the present invention. In order to use the evaluating method of the present invention, a user has to first activate the analyzing apparatus 1 (step S10). Next, the analyzing apparatus 1 enables the image capturing unit 12 to shoot a photo of a user according to the user's trigger (step S12), and the analyzing apparatus 1 further retrieves a face image of the user from the photo (step S14).

In the present invention, the analyzing apparatus 1 utilizes the processor 10 to control the image capturing unit 12 to shoot at least one photo, and the processor 10 executes an image analysis process to the photo to determine whether a face image covering the user's face is existed in the photo. In one embodiment, the processor 10 retrieves the face image from the photo for executing following analyzing and evaluation steps only if the face image is determined existed in the photo and the size of the face image is determined larger than a certain ratio of the entire photo (for example, exceeds 50% or 60% of the photo). If the face image is absent from the photo or existed in the photo without sufficient size, the processor 10 neither analyze nor evaluate the face image, and will control the display 11 to display a message for asking the user to re-take another photo.

In other embodiment, the analyzing apparatus 1 can receive at least one face image from a remote end through the wireless transmission unit 14 after being activated, and performs following analyzing and evaluation steps to the received face image. In this embodiment, it is unnecessary for the processor 10 to enable the image capturing unit 12.

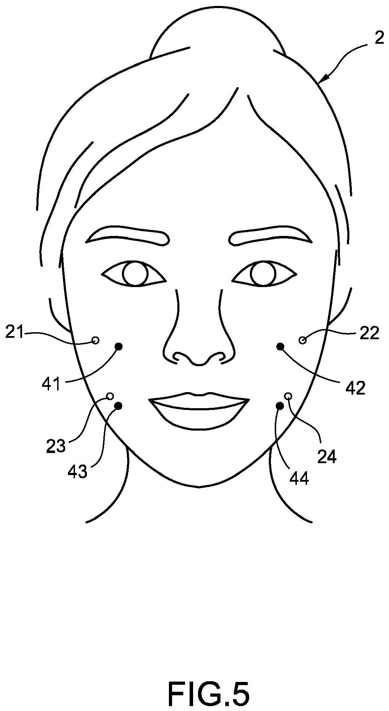

After the step S14, the processor 10 executes the analyzing algorithm 151 to perform an image analysis process to the retrieved or received face image, so as to obtain ideal positions of multiple facial muscle identifying points (referred to as the ideal facial muscle identifying points) from the face in the face image (step S16). In the present invention, the multiple ideal muscle identifying points (such as multiple ideal muscle identifying points 41-44 as shown in FIG. 5) are automatically generated by the analyzing algorithm 151 based on analyzing the five sense features of the face.

Specifically, parameters like human's five sense features and bones' positions, sizes, and distributed ratio on the face, etc., are unlikely changed due to external factors (such as climate) and time factor, which should be categorized as relatively stable features upon human's face. In the present invention, the analyzing algorithm 151 calculates the ideal positions of the multiple facial muscle identifying points of the face in the face image based on parameters such as positions, sizes, and distributed ratio of the five sense features of the face, in other words, if the user is well maintained, the positions of the multiple actual muscle identifying points 21-24 on the face of the user should be approximate to the positions of the multiple ideal muscle identifying points 41-44, or even overlapped with parts or all of the multiple ideal muscle identifying potions 41-44.

After the step S14, the processor 10 simultaneously identifies the retrieved or received face image through the fuzzy comparison algorithm 152 and the training model 153, so as to obtain actual potions of multiple facial muscle identifying points (i.e., the multiple actual muscle identifying points 21-24) from the face in the face image (step S18). In the present invention, the multiple actual muscle identifying points 21-24 are indicating the actual muscle status of the face in the face image.

In comparison with the five sense features and the bones, human's muscle is likely changed (for example, get drooping) due to external factors and time factor. In the present invention, the training model 153 records multiple reference images (which are multiple unspecified face images as discussed above), and these reference images are respectively pre-labeled, by a provider through manual manner, with multiple muscle identifying points thereon (which are considered as multiple actual muscle identifying points of a face covered in each of the reference images).

In the step S18, the processor 10 executes the fuzzy comparison algorithm 151 to perform a fuzzy comparison process to the face image with multiple reference images (not shown) of the training model 153 for obtaining at least one reference image out of the multiple reference images which is determined approximate to the face image. Eventually, the processor 10 deems the positions of multiple muscle identifying points labeled on the obtained reference image as the positions of the multiple actual muscle identifying points 21-24 on the face of the face image. For example, the processor 10 can retrieve the coordinates of the multiple labeled muscle identifying points from the obtained reference image, and directly set the positions of the multiple actual muscle identifying points 21-24 on the face of the face image according to the retrieved coordinates.

It can be seen that the fuzzy comparison algorithm 152 is different from the analyzing algorithm 151. In particular, the analyzing algorithm 151 utilizes the five sense features on the face to directly calculate the ideal positions of the multiple facial muscle identifying points. On the other hand, the fuzzy comparison algorithm 152 utilizes the pre-trained training model 153 to perform a fuzzy comparison process to the face image for obtaining the positions of the multiple actual muscle identifying points 21-24, which is different from the analyzing algorithm 151. More specific, the positions of the multiple actual muscle identifying points 21-24 obtained by the fuzzy comparison algorithm 152 are likely matching with user's current facial muscle status, i.e., the positions of the multiple actual muscle identifying points 21-24 can be used to represent the status of user's facial muscle such as tight or drooping.

In one embodiment, the processor 10 may first execute the step S16 and then execute the step S18, or vice versa. In other embodiment, the processor 10 may execute the step S16 and the step S18 simultaneously through multiplexing, the execution order is not limited thereto.

After the step S16 and the step S18, the processor 10 evaluates each of the actual muscle identifying points 21-24 respectively according to the multiple ideal muscle identifying points 41-44 in company with a pre-stored evaluation rule, and generates an evaluated result (step S20). In the present invention, the evaluated result can be textual descriptions (for example, "poor", "fair", "average", "good", "excellent", etc.) of scores (for example, "91-100 points", "81-90 points", "71-80 points", etc.) for each of the actual muscle identifying points 21-24, or the ratio (also called as performance rate) of each of the actual muscle identifying points 21-24 in comparison with each of the ideal muscle identifying points 41-44. However, the above description is only one of the exemplary embodiments of the present invention, not limited thereto.

In one embodiment, the evaluation rule is to apply a grid matrix onto the face image for calculating a distance between each of the actual muscle identifying points 21-24 and each of the ideal muscle identifying points 41-44 according to the grid matrix, so as to evaluate each of the actual muscle identifying points 21-24 based on the calculated distance. In other words, the evaluation rule uses each of the ideal muscle identifying points 41-44 as a foundational point and evaluates each of the actual muscle identifying points 21-24 based on such distance and foundational point.

After the step S20, the processor 10 controls the display 11 to display the aforementioned face image, multiple actual muscle identifying points 21-24, and multiple ideal muscle identifying points 41-44 (step S22), so the user can see the distances between the multiple actual muscle identifying points 21-24 and the multiple ideal muscle identifying points 41-44 directly on the display 11. Also, the processor 10 controls the display 11 to display the evaluated result (step S24), so the user can be aware of his/her current facial muscle status. In one embodiment, the processor 10 controls the display 11 to display the multiple actual muscle identifying points 21-24 as well as the multiple ideal muscle identifying points 41-44 overlapped with the face image, which applies a visible approach to directly show the distances between the multiple actual muscle identifying points 21-24 and the multiple ideal muscle identifying points 41-44 upon the face on the display 11.

FIG. 5 is a schematic diagram showing the comparison of multiple actual muscle identifying points and multiple ideal muscle identifying points according to a first embodiment of the present invention. After the step S16 as shown in FIG. 4, the processor 10 can obtain the aforementioned multiple ideal muscle identifying points 41-44 from the face of the user 2 in the face image. In the embodiment as shown in FIG. 5, the multiple ideal muscle identifying points 41-44 includes a first ideal muscle identifying point 41 corresponding to an ideal position of right side risorius muscle of the face, a second ideal muscle identifying point 42 corresponding to an ideal position of left side risorius muscle of the face, a third ideal muscle identifying point 43 corresponding to an ideal position of right side masticatory muscle of the face, and a fourth ideal muscle identifying point 44 corresponding to an ideal position of left side masticatory muscle of the face.

After the step S18 as shown in FIG. 4, the processor 10 can identify the aforementioned multiple actual muscle identifying points 21-24 from the face of the user 2 in the face image. In the step S20 as shown in FIG. 4, the processor 10 compares a first actual muscle identifying point 21 which corresponds to an actual position of right side risorius muscle of the face with the first ideal muscle identifying point 41, compares a second actual muscle identifying point 22 which corresponds to an actual position of left side risorius muscle of the face with the second ideal muscle identifying point 42, compares a third actual muscle identifying point 23 which corresponds to an actual position of right side masticatory muscle of the face with the third ideal muscle identifying point 43, and compares a fourth actual muscle identifying point 24 which corresponds to an actual position of left side masticatory muscle of the face with the fourth ideal muscle identifying point 44.

After the above discussed actions are executed completely, the processor 10 can generate at least four evaluation results, wherein the at least four evaluation results include a first evaluation result representing the distance between the first actual muscle identifying point 21 and the first ideal muscle identifying point 41, a second evaluation result representing the distance between the second actual muscle identifying point 22 and the second ideal muscle identifying point 42, a third evaluation result representing the distance between the third actual muscle identifying point 23 and the third ideal muscle identifying point 43, and a fourth evaluation result representing the distance between the fourth actual muscle identifying point 24 and the fourth ideal muscle identifying point 44. As a result, the user can be clearly aware of the difference between his/her current facial muscle status and an ideal muscle status recommended to his/her face, so as to determine whether the currently applied care products, cosmetics, or maintenance manners are effective or not.

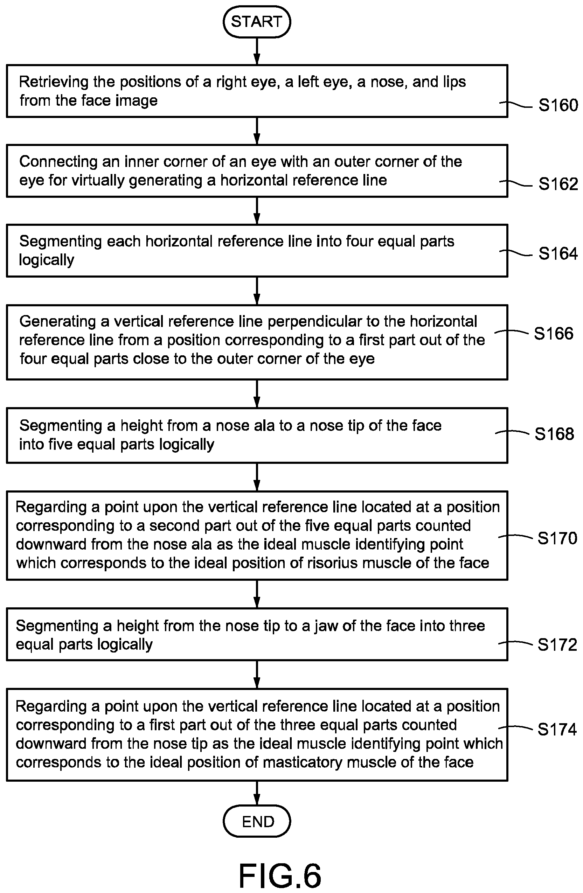

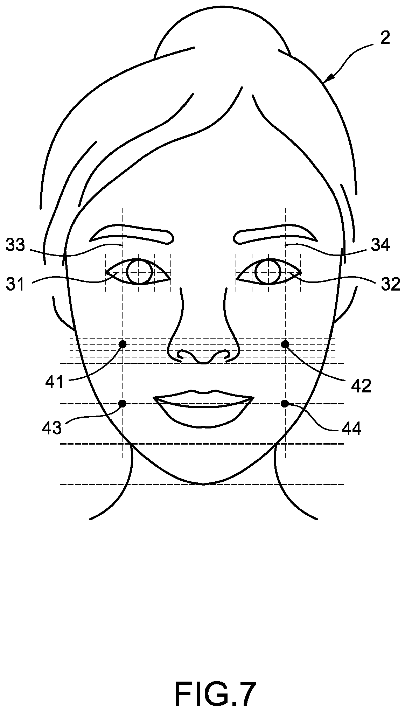

As discussed, the analyzing algorithm 151 in the step S16 of FIG. 4 executes the calculation based on the actual five sense features on the face of the face image, and the purpose of executing the step S16 is to determine the ideal positions where risorius muscle and masticatory muscle supposed to be on the face of the user in the face image, i.e., to determine the positions of the first to fourth ideal muscle identifying points 41-44 for the face. In the following paragraphs, FIG. 6 and FIG. 7 are provided to detailed describe the calculation logic of the analyzing algorithm 151 in performing such calculation.

Please refer to FIG. 6 and FIG. 7, where FIG. 6 is a flowchart for analyzing multiple ideal muscle identifying points according to a first embodiment of the present invention, and FIG. 7 is a schematic diagram showing multiple ideal muscle identifying points according to a first embodiment of the present invention. In the following paragraphs, FIG. 6 and FIG. 7 are used to more specifically describe the step S16 of FIG. 4.

In the present invention, the processor 10 performs an image identification process to the face image after obtaining the face image, so as to retrieve at least the positions of right eye, left eye, nose, and lips (step S160). In one embodiment, the processor 10 utilizes Dlib Face Landmark system to perform the image identification process to the face image for obtaining the positions of right eye, left eye, nose, and lips from the face in the face image.

Specifically, the Dlib Face Landmark system can identify outstanding points from a face (around 119 points), and constitutes outstanding features of the face, such as eyebrows, eyes, nose, lips, contours of cheeks, contour of jaw, etc., according to these identified outstanding points. By performing the image identification process to the face image through the Dlib Face Landmark system, the processor 10 can obtain one or more outstanding features which are essential for the upcoming analysis from the face in the face image.

After the step S160, the processor 10 connects an inner corner of an eye with an outer corner of the eye for virtually generating a reference line (step S162). In particular, as shown in FIG. 7, the processor 10 in the step S162 is to virtually generate a first horizontal reference line 31 through connecting an inner corner of a right eye with an outer corner of the right eye and a second horizontal reference line 32 through connecting an inner corner of a left eye with an outer corner of the left eye.

Next, the processor 10 respectively segments each of the reference lines into four equal parts logically (step S164), and respectively generates another reference line perpendicular to such reference line from a position corresponding to a first part out of the four equal parts which is close to the outer corner of the eye (step S166). In particular, as shown in FIG. 7, the processor 10 logically segments the first horizontal reference line 31 into four equal parts, and generates a first vertical reference line 33 perpendicular to the first horizontal reference line 31 from a position corresponding to a first part out of the four equal parts which is close to the outer corner of the right eye. Also, the processor 10 logically segments the second horizontal reference line 32 into four equal parts, and generates a second vertical reference line 34 perpendicular to the second horizontal reference line 32 from a position corresponding to a first part out of the four equal parts which is close to the outer corner of the left eye.

Next, the processor 10 logically segments a height from the nose ala (e.g., a position close to an outer side of the nose ala) to the nose tip of the face into five equal parts (step S168), and regards a point upon each of the vertical reference lines which is located at a position corresponding to a second part out of the five equal parts counted downward from the nose ala as the ideal muscle identifying point which corresponds to the ideal position of risorius muscle of the face (step S170).

In particular, as shown in FIG. 7, the processor 10 obtains a first point upon the first vertical reference line 33 which locates at a position corresponding to the second part out of the five equal parts counted downward from the nose ala, and regards the first point as the first ideal muscle identifying point 41 which represents the ideal position of right side risorius muscle of the face. Also, the processor 10 obtains a second point upon the second vertical reference line 34 which locates at a position corresponding to the second part out of the five equal parts counted downward from the nose ala, and regards the second point as the second ideal muscle identifying point 42 which represents the ideal position of left side risorius muscle of the face.

Next, the processor 10 logically segments a height from the nose tip to the jaw (e.g., the outline of the jaw) into three equal parts (step S172), and regards a point upon each of the vertical reference lines which is located at a position corresponding to a first part out of the three equal parts counted downward from the nose tip as the ideal muscle identifying point which corresponds to the ideal position of masticatory muscle of the face (step S174).

In particular, as shown in FIG. 7, the processor 10 obtains a third point upon the first vertical reference line 33 which locates at a position corresponding to the first part out of the three equal parts counted downward from the nose tip, and regards the third point as the third ideal muscle identifying point 43 which represents the ideal position of right side masticatory muscle of the face. Also, the processor 10 obtains a fourth point upon the second vertical reference line 34 which locates at a position corresponding to the first part out of the three equal parts counted downward from the nose tip, and regards the fourth point as the fourth ideal muscle identifying point 44 which represents the ideal position of left side masticatory muscle of the face.

It is worth saying that the processor 10 in the aforementioned embodiment is to first calculate the first ideal muscle identifying point 41 and the third ideal muscle identifying point 43 at right side of the face in the face image, and then calculates the second ideal muscle identifying point 42 and the fourth ideal muscle identifying point 44 at left side of the face in the face image. In other embodiment, however, the processor 10 may first calculate the first ideal muscle identifying point 41 and the second ideal muscle identifying point 42 corresponding to risorius muscle of the face, and then calculates the third ideal muscle identifying point 43 and the fourth ideal muscle identifying point 44 corresponding to masticatory muscle of the face. Furthermore, the processor 10 may calculate the four ideal muscle identifying points 41-44 simultaneously through multiplexing, not limited to the above embodiments.

As shown in FIG. 6 and FIG. 7, the multiple ideal muscle identifying points 41-44 in the present invention are calculated based on relatively stable features, such as the eyes, the nose, and the jaw of the face in the face image, so these points can be regarded as reasonable references of the ideal positions of risorius muscle and masticatory muscle of user's face. Therefore, the processor 10 can regard these ideal muscle identifying points 41-44 as evaluation foundations, and uses these ideal muscle identifying points 41-44 to evaluate user's current facial muscle status, i.e., analyzes the distances between the actual positions and the ideal positions of the multiple muscle identifying points, and evaluates the facial muscle status according to the analyzed distances.

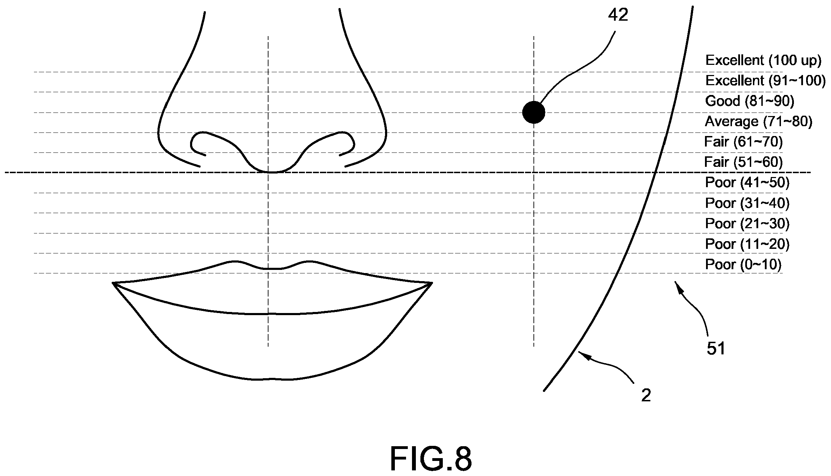

Please refer to FIG. 8, which is a schematic diagram showing an evaluation rule according to a first embodiment of the present invention. When performing the evaluation process, the processor 10 in one of the exemplary embodiments, applies a grid matrix 51 onto the face image according to the aforementioned evaluation rule for logically segmenting the entire face image into multiple blocks, wherein the grid matrix 51 indicates multiple intervals with same size. By doing so, the processor 10 is allowed to calculate the distance between each of the actual muscle identifying points 21-24 and each of the ideal muscle identifying points 41-44 by counting the blocks.

In one embodiment, the processor 10 evaluates the status of the first actual muscle identifying point 21 and the second actual muscle identifying point 22 as "average" when the distances between the positions of the two actual muscle identifying points 21-22 and the positions of the first ideal muscle identifying point 41 and the second ideal muscle identifying point 42 are smaller than or equal to a threshold (for example, smaller than a height of one black), evaluates the status of the first actual muscle identifying point 21 and the second actual muscle identifying point 22 as "good" or "excellent" when the positions of the two actual muscle identifying points 21-22 are higher than the positions of the first ideal muscle identifying point 41 and the second ideal muscle identifying point 42 and the distances between the positions of the two actual muscle identifying points 21-22 and the positions of the two ideal muscle identifying points 41-42 are bigger than the threshold. For example, the processor 10 can evaluate the status of the two actual muscle identifying points 21-22 as "good" if the positions of the two actual muscle identifying points 21-22 are higher than the positions of the two ideal muscle identifying points 41-42 by a height of one block, and evaluates the status of the two actual muscle identifying points 21-22 as "excellent" if the positions of the two actual muscle identifying points 21-22 are higher than the positions of the two ideal muscle identifying points 41-42 by a height of at least two blocks.

Further, the processor 10 can evaluate the status of the first actual muscle identifying point 21 and the second actual muscle identifying point 22 as "fair" or "poor" when the positions of the two actual muscle identifying points 21-22 are lower than the positions of the first ideal muscle identifying point 41 and the second ideal muscle identifying point 42 and the distances between the positions of the two actual muscle identifying points 21-22 and the positions of the two ideal muscle identifying points 41-42 are bigger than the threshold. For example, the processor 10 can evaluate the status of the two actual muscle identifying points 21-22 as "fair" if the positions of the two actual muscle identifying points 21-22 are lower than the positions of the two ideal muscle identifying point 41-42 by a height of one to two blocks, and evaluates the status of the two actual muscle identifying points 21-22 as "poor" if the positions of the two actual muscle identifying points 21-22 are lower than the positions of the two ideal muscle identifying points 41-42 by a height of three or more blocks.

In other embodiment as shown in FIG. 8, the processor 10 logically segments the height from the nose tip to the nose ala of the face in the face image into five equal parts according to the evaluation rule, and utilizes the intervals of these five equal parts as the aforementioned grid matrix 51, i.e., each interval of the five equal parts is regarded as one block of the grid matrix 51 as discussed above.

In this embodiment, the processor 10 can evaluate the status of the two actual muscle identifying points 21-22 as "average" if the distances between the two actual muscle identifying points 21-22 and the two ideal muscle identifying points 41-42 are smaller than or equal to one part out of the five equal parts, evaluates the status of the two actual muscle identifying points 21-22 as "good" if the two actual muscle identifying points 21-22 are higher than the two ideal muscle identifying points 41-42 by one part out of the five equal parts, evaluates the status of the two actual muscle identifying points 21-22 as "excellent" if the two actual muscle identifying points 21-22 are higher than the two ideal muscle identifying points 41-42 by at least two parts out of the five equal parts, evaluates the status of the two actual muscle identifying points 21-22 as "fair" if the two actual muscle identifying points 21-22 are lower than the two ideal muscle identifying points 41-42 by one to two parts out of the five equal parts, and evaluates the status of the two actual muscle identifying points 21-22 as "poor" if the two actual muscle identifying points 21-22 are lower than the two ideal muscle identifying points 41-42 by three or more parts out of the five equal parts.

However, the above descriptions are only a part of the exemplary embodiments of the present invention, not limited thereto.

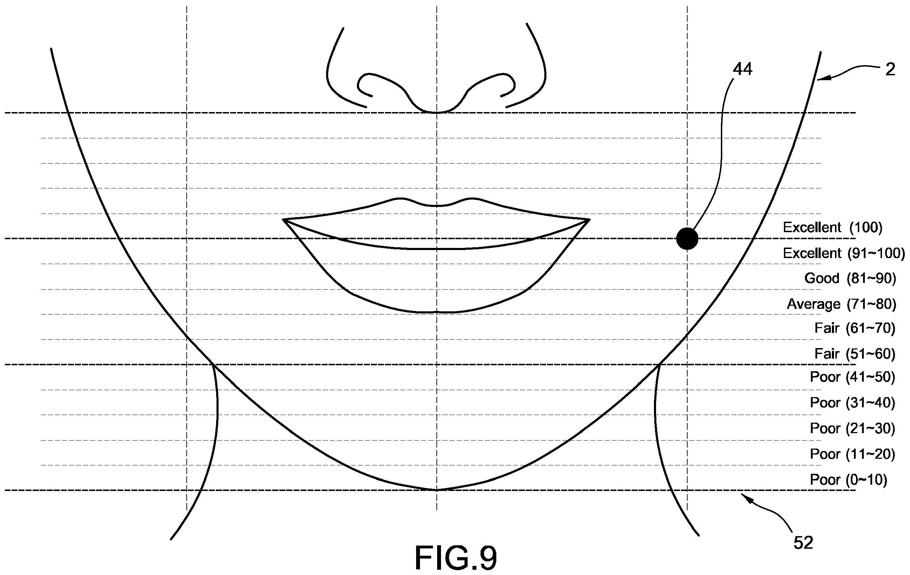

Please refer to FIG. 9, which is a schematic diagram showing an evaluation rule according to a second embodiment of the present invention. When evaluating the third actual muscle identifying point 23 and the fourth actual muscle identifying point 24 which correspond to the positions of masticatory muscle of the face in the face image, the processor 10 is mainly applying another grid matrix 52 onto the face image according to the aforementioned evaluation rule for logically segmenting the entire face image into multiple blocks, wherein the grid matrix 52 indicates multiple intervals with same size. It is worth saying that the third and fourth actual muscle identifying points 23-24 are located at the lower part of the face image, when performing such logical segment process, the processor 10 can only segment the lower part of the face image (for example, only to the part below the nose of the face) so as to save system resources and improve processing efficiency.

In this embodiment, the size of each of the intervals of the grid matrix 52 is identical to or different from that of the aforementioned grid matrix 51, not limited thereto.

In this embodiment, the processor 10 can evaluate the status of the third actual muscle identifying point 23 and the fourth actual muscle identifying point 24 as "excellent" if the distances between the positions of the two actual muscle identifying points 23-24 and the positions of the third ideal muscle identifying point 43 as well as the fourth ideal muscle identifying point 44 are smaller than or equal to a threshold (e.g., smaller than a height of one block). Also, the processor 10 can evaluate the status of the third actual muscle identifying point 23 and the fourth actual muscle identifying point 24 as "average", "fair", or "poor" in an order if the positions of the two actual muscle identifying points 23-24 are lower than the positions of the two ideal muscle identifying points 43-44 by different distances larger than the threshold.

For example, the processor 10 can evaluate the status of the third actual muscle identifying point 23 and the fourth actual muscle identifying point 24 as "good" if the positions of the two actual muscle identifying points 23-24 are lower than the positions of the two ideal muscle identifying points 43-44 by a hieght of one block, evaluates the status of the third actual muscle identifying point 23 and the fourth actual muscle identifying point 24 as "average" if the positions of the two actual muscle identifying points 23-24 are lower than the positions of the two ideal muscle identifying points 43-44 by a height of two blocks, evaluates the status of the third actual muscle identifying point 23 and the fourth actual muscle identifying point 24 as "fair" if the positions of the two actual muscle identifying points 23-24 are lower than the positions of the two ideal muscle identifying points 43-44 by a height of three to four blocks, and evaluates the status of the third actual muscle identifying point 23 and the fourth actual muscle identifying point 24 as "poor" if the positions of the two actual muscle identifying points 23-24 are lower than the positions of the two ideal muscle identifying points 43-44 by a height of five or more blocks.

In other embodiment as shown in FIG. 9, the processor 10 logically segments the height from the nose tip to the jaw of the face in the face image into fifteen equal parts according to the evaluation rule, and utilizes the intervals of these fifteen equal parts as the aforementioned grid matrix 52, i.e., each of the intervals of the fifteen equal parts is regarded as one block of the grid matrix 52 as discussed above.

In this embodiment, the processor 10 can evaluate the status of the two actual muscle identifying points 23-24 as "excellent" if the distances between the two actual muscle identifying points 23-24 and the two ideal muscle identifying points 43-44 are smaller than or equal to one part out of the fifteen equal parts, evaluates the status of the two actual muscle identifying points 23-24 as "good" if the two actual muscle identifying points 23-24 are lower than the two ideal muscle identifying points 43-44 by at least one part out of the fifteen equal parts, evaluates the status of the two actual muscle identifying points 23-24 as "average" if the two actual muscle identifying points 23-24 are lower than the two ideal muscle identifying points 43-44 by at least two parts out of the fifteen equal parts, evaluates the status of the two actual muscle identifying points 23-24 as "fair" if the two actual muscle identifying points 23-24 are lower than the two ideal muscle identifying points 43-44 by three to four parts out of the fifteen equal parts, and evaluates the status of the two actual muscle identifying points 23-24 as "poor" if the two actual muscle identifying points 23-24 are lower than the two ideal muscle identifying points 43-44 by five or more parts out of the fifteen equal parts.

However, the above description is only one of the exemplary embodiments of the present invention, not limited thereto.

Throughout the aforementioned evaluation rule, the processor 10 can instantly and accurately evaluate the facial muscle status of the user 2 right after the multiple actual muscle identifying points 21-24 as well as the multiple ideal muscle identifying points 41-44 are obtained from the face of the face image.

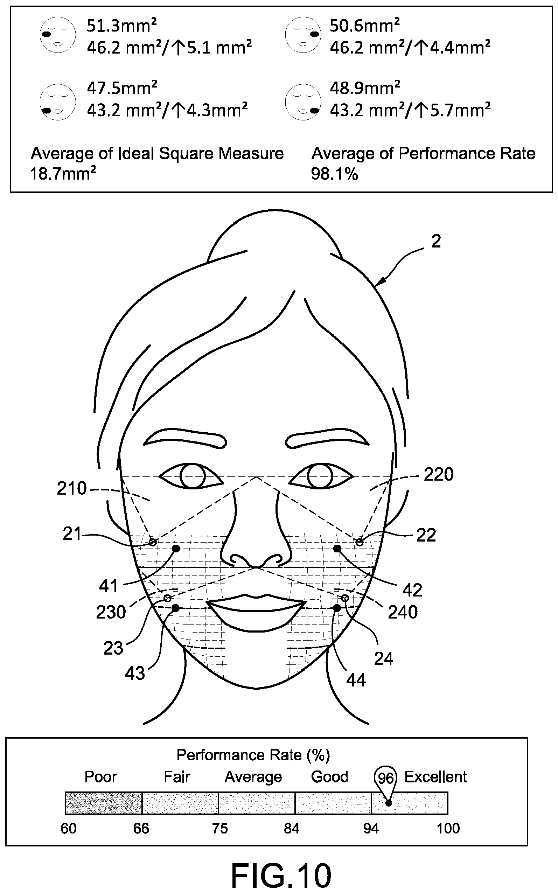

Please refer to FIG. 10, which is a schematic diagram showing an evaluated result according to a first embodiment of the present invention.

As disclosed in FIG. 10, after obtaining the multiple actual muscle identifying points 21-24 as well as the multiple ideal muscle identifying points 41-44, the processor 10 overlaps the face image of the user 2, the multiple actual muscle identifying points 21-24 and the multiple ideal muscle identifying points 41-44 and displays the overlapped result on the display 11, so the user 2 can be aware of the distance between his/her current facial muscle status and an ideal muscle status.

In the present invention, the processor 10 can calculate a square measure of a first triangular region 210 virtually constituted by the first actual muscle identifying point 21, the position of nasion, and the position of right side temple of the face in the face image (exampled as 51.3 mm² in FIG. 10), calculates a square measure of a second triangular region 220 virtually constituted by the second actual muscle identifying point 22, the position of nasion, and the position of left side temple of the face in the face image (exampled as 50.6 mm² in FIG. 10), calculates a square measure of a third triangular region 230 virtually constituted by the third actual muscle identifying point 23, the position of nose tip, and the position of right side cheek of the face in the face image (exampled as 47.5 mm² in FIG. 10), and calculates a square measure of a fourth triangular region 240 virtually constituted by the fourth actual muscle identifying point 24, the position of nose tip, and the position of left side cheek of the face in the face image (exampled as 48.9 mm² in FIG. 10).

In one embodiment, the processor 10 can further calculate a square measure of a first ideal triangular region (not shown in the FIGs) virtually constituted by the first ideal muscle identifying point 41, the position of nasion, and the position of right side temple of the face (e.g., 46.2 mm² as shown in FIG. 10), calculates a square measure of a second ideal triangular region (not shown in the FIGs) virtually constituted by the second ideal muscle identifying point 42, the position of nasion, and the position of left side temple of the face (e.g., 46.2 mm² as shown in FIG. 10), calculates a square measure of a third ideal triangular region (not shown in the FIGs) virtually constituted by the third ideal muscle identifying point 43, the position of nose tip, and the position of right side cheek of the face (e.g., 43.2 mm² as shown in FIG. 10), and calculates a square measure of a fourth ideal triangular region (not shown in the FIGs) virtually constituted by the fourth ideal muscle identifying point 44, the position of nose tip, and the position of left side cheek of the face (e.g., 43.2 mm² as shown in FIG. 10).

As discussed above, within a certain range, the status of user's facial muscle will be evaluated better once the square measures of the triangular regions 210-240 are getting smaller. Besides, the processor 10 can calculate the distances between square measure of each of the triangular regions 210-240 and the square measure of each of the ideal triangular regions, so as to further calculate a performance rate corresponding to the maintenance manner(s) currently applied by the user. In other words, once the square measure of each of the triangular regions 210-240 is determined approximate to the square measure of each of the ideal triangular regions, it means the performance rate of the maintenance manners applied by the user is getting higher.

By utilizing the technical solution provided in the disclosures, a user can be aware of the evaluation of his/her current facial muscle status in comparison with an ideal one, so as to determine whether the currently applied care products/cosmetics/maintenance manners are effective or not, which is convenient and irreplaceable.

## Claims

1. A method for analyzing and evaluating facial muscle status, applied to a face image analyzing apparatus (1) having a processor (10), an image capturing unit (12), and a display (11), comprising following steps of:
a) taking a photo of a user (2) by the image capturing unit (12);
b) retrieving a face image of the user (2) from the photo;
c) executing an analyzing algorithm (151) by the processor (10) to analyze the face image for obtaining multiple ideal muscle identifying points (41-44) from the face image, wherein the multiple ideal muscle identifying points (41-44) are automatically generated based on five sense features on a face in the face image;
d) identifying the face image through a fuzzy comparison algorithm (152) in company with a training model (153) by the processor (10) for obtaining multiple actual muscle identifying points (21-24) from the face in the face image, wherein the multiple actual muscle identifying points (21-24) are corresponding to an actual muscle status of the face;
e) evaluating each of the multiple actual muscle identifying points (21-24) by the processor (10) according to each of the multiple ideal muscle identifying points (41-44) and an evaluation rule for generating an evaluated result;
f) overlapping and displaying the face image, the multiple ideal muscle identifying points (41-44), and the multiple actual muscle identifying points (21-24) on the display (11); and
g) displaying the evaluated result on the display (11).

2. The method in claim 1, wherein the multiple ideal muscle identifying points (41-44) comprise a first ideal muscle identifying point (41) corresponding to an ideal position of right side risorius muscle of the face, a second ideal muscle identifying point (42) corresponding to an ideal position of left side risorius muscle of the face, a third ideal muscle identifying point (43) corresponding to an ideal position of right side masticatory muscle of the face, and a fourth ideal muscle identifying point (44) corresponding to an ideal position of left side masticatory muscle of the face, and the multiple actual muscle identifying points (21-24) comprise a first actual muscle identifying point (21) corresponding to an actual position of right side risorius muscle of the face, a second actual muscle identifying point (22) corresponding to an actual position of left side risorius muscle of the face, a third actual muscle identifying point (23) corresponding to an actual position of right side masticatory muscle of the face, and a fourth actual muscle identifying point (24) corresponding to an actual position of left side masticatory risorius muscle of the face.

3. The method in claim 1, wherein the training model (153) records multiple reference images respectively labeled with multiple muscle identifying points thereon, the step d) is to perform a fuzzy comparison process to the face image and the multiple reference images of the training model (153) by the fuzzy comparison algorithm (152) for obtaining at least one of the reference images which is determined approximate to the face image, and sets the positions of the multiple actual muscle identifying points (21-24) on the face of the face image according to the positions of the multiple muscle identifying points labeled on the obtained reference image.

4. The method in claim 2, wherein the step c) comprises following steps of:
c11) identifying the face of the face image for at least obtaining the positions of a right eye, a left eye, a nose, and lips;
c12) virtually connecting an inner corner of the right eye with an outer corner of the right eye for generating a first horizontal reference line (31);
c13) logically segmenting the first horizontal reference line (31) into four equal parts;
c14) virtually generating a first vertical reference line (33) perpendicular to the first horizontal reference line (31) from a position corresponding to a first part out of the four equal parts which is close to the outer corner of the right eye;
c15) logically segmenting a height from a nose ala of the nose to a nose tip of the nose into five equal parts;
c16) regarding a point upon the first vertical reference line (33) located at a position corresponding to a second part out of the five equal parts counted downward from the nose ala as the first ideal muscle identifying point (41);
c17) logically segmenting a height from the nose tip to a jaw of the face into three equal parts; and
c18) regarding a point upon the first vertical reference line (33) located at a position corresponding to a first part out of the three equal parts counted downward from the nose tip as the third ideal muscle identifying point (43).

5. The method in claim 4, wherein the step c11) is to identify the face image through Dlib Face Landmark system for obtaining the positions of the right eye, the left eye, the nose, and the lips.

6. The method in claim 2, wherein the step c) comprises following steps of:
c21) identifying the face of the face image for at least obtaining the positions of a right eye, a left eye, a nose, and lips;
c22) virtually connecting an inner corner of the left eye with an outer corner of the left eye for generating a second horizontal reference line (32);
c23) logically segmenting the second horizontal reference line (32) into four equal parts;
c24) virtually generating a second vertical reference line (34) perpendicular to the second horizontal reference line (32) from a position corresponding to a first part out of the four equal parts which is close to the outer corner of the left eye;
c25) logically segmenting a height from a nose ala of the nose to a nose tip of the nose into five equal parts;
c26) regarding a point upon the second vertical reference line (34) located at a position corresponding to a second part out of the five equal parts counted downward from the nose ala as the second ideal muscle identifying point (42);
c27) logically segmenting a height from the nose tip to a jaw of the face into three equal parts; and
c28) regarding a point upon the second vertical reference line (34) located at a position corresponding to a first part out of the three equal parts counted downward from the nose tip as the fourth ideal muscle identifying point (44).

7. The method in claim 6, wherein the step c21) is to identify the face image through Dlib Face Landmark system for obtaining the positions of the right eye, the left eye, the nose, and the lips.

8. The method in claim 2, wherein in the step e), the processor (10) evaluates the status of the first actual muscle identifying point (21) and the second actual muscle identifying point (22) as average if the distances between the positions of the first actual muscle identifying point (21) and the second actual muscle identifying point (22) and the positions of the first ideal muscle identifying point (41) and the second ideal muscle identifying point (42) are smaller than or equal to a threshold, evaluates the status of the first actual muscle identifying point (21) and the second actual muscle identifying point (22) as good or excellent if the positions of the first actual muscle identifying point (21) and the second actual muscle identifying point (22) are higher than the positions of the first ideal muscle identifying point (41) and the second ideal muscle identifying point (42) and the distances between the positions of the first actual muscle identifying point (21) and the second actual muscle identifying point (22) and the positions of the first ideal muscle identifying point (41) and the second ideal muscle identifying point (42) are bigger than the threshold, and evaluates the status of the first actual muscle identifying point (21) and the second actual muscle identifying point (22) as fair or poor if the positions of the first actual muscle identifying point (21) and the second actual muscle identifying point (22) are lower than the positions of the first ideal muscle identifying point (41) and the second ideal muscle identifying point (42) and the distances between the positions of the first actual muscle identifying point (21) and the second actual muscle identifying point (22) and the positions of the first ideal muscle identifying point (41) and the second ideal muscle identifying point (42) are bigger than the threshold.

9. The method in claim 2, wherein in the step e), the processor (10) evaluates the status of the third actual muscle identifying point (23) and the fourth actual muscle identifying point (24) as excellent if the distances between the positions of the third actual muscle identifying point (23) and the fourth actual muscle identifying point (24) and the positions of the third ideal muscle identifying point (43) and the fourth ideal muscle identifying point (44) are smaller than or equal to a threshold, and evaluates the status of the third actual muscle identifying point (23) and the fourth actual muscle identifying point (24) as average, fair, or poor in an order if the positions of the third actual muscle identifying point (23) and the fourth actual muscle identifying point (24) are lower than the positions of the third ideal muscle identifying point (43) and the fourth ideal muscle identifying point (44) and the distances between the positions of the third actual muscle identifying point (23) and the fourth actual muscle identifying point (24) and the positions of the third ideal muscle identifying point (43) and the fourth ideal muscle identifying point (44) are bigger than the threshold.

10. The method in claim 2, wherein the evaluation rule is logically segmenting a height from the position of a nose tip to the position of a nose ala of the face in the face image into five equal parts, and evaluates the status of the first actual muscle identifying point (21) and the second actual muscle identifying point (22) as average if the distance between the positions of the first actual muscle identifying point (21) and the second actual muscle identifying point (22) and the positions of the first ideal muscle identifying point (41) and the second ideal muscle identifying point (42) are smaller than or equal to one part out of the five equal parts, evaluates the status of the first actual muscle identifying point (21) and the second actual muscle identifying point (22) as good if the positions of the first actual muscle identifying point (21) and the second actual muscle identifying point (22) are higher than the positions of the first ideal muscle identifying point (41) and the second ideal muscle identifying point (42) by one part out of the five equal parts, evaluates the status of the first actual muscle identifying point (21) and the second actual muscle identifying point (22) as excellent if the positions of the first actual muscle identifying point (21) and the second actual muscle identifying point (22) are higher than the positions of the first ideal muscle identifying point (41) and the second ideal muscle identifying point (42) by two parts out of the five equal parts, evaluates the status of the first actual muscle identifying point (21) and the second actual muscle identifying point (22) as fair if the positions of the first actual muscle identifying point (21) and the second actual muscle identifying point (22) are lower than the positions of the first ideal muscle identifying point (41) and the second ideal muscle identifying point (42) by one to two parts out of the five equal parts, and evaluates the status of the first actual muscle identifying point (21) and the second actual muscle identifying point (22) as poor if the positions of the first actual muscle identifying point (21) and the second actual muscle identifying point (22) are lower than the positions of the first ideal muscle identifying point (41) and the second ideal muscle identifying point (42) by three or more parts out of the five equal parts.

11. The method in claim 2, wherein the evaluation rule is logically segmenting a height from the position of a nose tip to the position of a jaw of the face in the face image into fifteen equal parts, and evaluates the status of the third actual muscle identifying point (23) and the fourth actual muscle identifying point (24) as excellent if the distances between the positions of the third actual muscle identifying point (23) and the fourth actual muscle identifying point (24) and the positions of the third ideal muscle identifying point (43) and the fourth ideal muscle identifying point (44) are smaller than or equal to one part out of the fifteen equal parts, evaluates the status of the third actual muscle identifying point (23) and the fourth actual muscle identifying point (24) as good if the positions of the third actual muscle identifying point (23) and the fourth actual muscle identifying point (24) are lower than the positions of the third ideal muscle identifying point (43) and the fourth ideal muscle identifying point (44) by at least one part out of the fifteen equal parts, evaluates the status of the third actual muscle identifying point (23) and the fourth actual muscle identifying point (24) as average if the positions of the third actual muscle identifying point (23) and the fourth actual muscle identifying point (24) are lower than the positions of the third ideal muscle identifying point (43) and the fourth ideal muscle identifying point (44) by at least two parts out of the fifteen equal parts, evaluates the status of the third actual muscle identifying point (23) and the fourth actual muscle identifying point (24) as fair if the positions of the third actual muscle identifying point (23) and the fourth actual muscle identifying point (24) are lower than the positions of the third ideal muscle identifying point (43) and the fourth ideal muscle identifying point (44) by three to four parts out of the fifteen equal parts, and evaluates the status of the third actual muscle identifying point (23) and the fourth actual muscle identifying point (24) as poor if the positions of the third actual muscle identifying point (23) and the fourth actual muscle identifying point (24) are lower than the positions of the third ideal muscle identifying point (43) and the fourth ideal muscle identifying point (44) by five or more parts out of the fifteen equal parts.

12. The method in claim 2, wherein the evaluated result comprises textual descriptions or scores of each of the multiple actual muscle identifying points (21-24), or ratio of each of the multiple actual muscle identifying points (21-24) in comparison with each of the multiple ideal muscle identifying points (41-44).

13. The method in claim 2, further comprising following steps:
h1) simulating a first virtual triangle (210) constituted by the first actual muscle identifying point (21), a position of nasion of the face in the face image, and a position of right side temple of the face in the face image by the processor (10);
h2) simulating a second virtual triangle (220) constituted by the second actual muscle identifying point (22), the position of nasion of the face, and a position of left side temple of the face in the face image by the processor (10);
h3) simulating a third virtual triangle (230) constituted by the third actual muscle identifying point (23), a position of nose tip of the face in the face image, and a position of right side cheek of the face in the face image by the processor (10);
h4) simulating a fourth virtual triangle (240) constituted by the fourth actual muscle identifying point (24), the position of nose tip of the face, and a position of left side cheek of the face in the face image by the processor (10);
wherein, square measures of the first virtual triangle (210), the second virtual triangle (220), the third virtual triangle (230), and the fourth virtual triangle (240) are inversely proportional to the muscle status of the user.
